# EUROPEAN PATENT SPECIFICATION

(11) **EP 4 186 409 B1**
(45) Date of publication and mention of the grant of the patent: **25.11.2026**
(21) Application number: 21850153.4
(22) Date of filing: 30.07.2021
(51) Int. Cl.: A61B 1/00, A61B 1/04, A61B 1/06, A61B 5/00, G06T 7/00, G16H 20/40, G16H 30/40, G16H 50/20

(54) **IMAGE PROCESSING DEVICE, IMAGE PROCESSING METHOD, IMAGE PROCESSING PROGRAM, ENDOSCOPE DEVICE, AND ENDOSCOPE IMAGE PROCESSING SYSTEM**
BILDVERARBEITUNGSVORRICHTUNG, BILDVERARBEITUNGSVERFAHREN, BILDVERARBEITUNGSPROGRAMM, ENDOSKOPVORRICHTUNG UND ENDOSKOPBILDVERARBEITUNGSSYSTEM
DISPOSITIF DE TRAITEMENT D'IMAGE, PROCÉDÉ DE TRAITEMENT D'IMAGE, PROGRAMME DE TRAITEMENT D'IMAGE, DISPOSITIF D'ENDOSCOPE ET SYSTÈME DE TRAITEMENT D'IMAGE D'ENDOSCOPE

(30) Priority: 31.07.2020 JP 2020130535
(43) Date of publication of application: 31.05.2023
(73) Proprietor: Tokyo University of Science Foundation, Tokyo 162-8601 (JP); RIKEN, Wako-shi, Saitama 351-0198 (JP)
(72) Inventor: TAKEMURA, Hiroshi, Tokyo 162-8601 (JP); SOGA, Kohei, Tokyo 162-8601 (JP); IKE, Reiichirou, Tokyo 162-8601 (JP); TAKAMATSU, Toshihiro, Tokyo 162-8601 (JP); IKEMATSU, Hiroaki, Tokyo 104-0045 (JP); YOKOTA, Hideo, Wako-shi, Saitama 351-0198 (JP)
(74) Representative: Berggren Oy
(86) International application number: PCT/JP2021/028496
(87) International publication number: WO 2022/025290

(56) References cited:
- WO-A1-2014/192876
- WO-A1-2020/105699
- JP-A- 2009 300 131
- JP-A- 2009 300 131
- JP-A- 2011 083 486
- JP-A- 2011 092 220
- US-A1- 2022 020 496
- HAMED AKBARI ET AL: "Cancer detection using infrared hyperspectral imaging", CANCER SCIENCE, vol. 102, no. 4, 4 January 2011 (2011-01-04), JP, pages 852 - 857, XP055503624, ISSN: 1347-9032, DOI: 10.1111/j.1349-7006.2011.01849.x
- HAMED AKBARI, UTO KUNIAKI, KOSUGI YUKIO, KOJIMA KAZUYUKI, TANAKA NAOFUMI: "Cancer detection using infrared hyperspectral imaging", CANCER SCIENCE, JAPANESE CANCER ASSOCIATION, TOKYO, JP, vol. 102, no. 4, 1 April 2011 (2011-04-01), JP , pages 852 - 857, XP055503624, ISSN: 1347-9032, DOI: 10.1111/j.1349-7006.2011.01849.x

## Description

### Technical Field

The technology of the present disclosure relates to an image processing device, an image processing method, an image processing program, an endoscope device, and an endoscope image processing system.

### Background Art

Conventionally, a biological tissue identification device that identifies normality/abnormality of biological tissue is known (for example, see Japanese Patent Application Laid-Open (JP-A) No. 2009-300131.). The biological tissue identification device of JP-A No. 2009-300131 performs irradiation in a wavelength range of 900 nm to 1700 nm (paragraph [0025]), and identifies normality/abnormality of biological tissue based on a spectral distribution curve in a range of 1200 to 1320 nm (paragraph [0021]). This biological tissue identification device targets an inner wall of gastric cancer which is the surface of biological tissue (paragraph [0035]).

In addition, there is known a biological examination device that evaluates a change in spectral shape between a cancer cell and a normal cell that occurs in a wavelength range of 1510 nm to 1530 nm and/or a wavelength range of 1480 nm to 1500 nm in a spectrum obtained by irradiating biological tissue with near-infrared light, by quantifying the change as a secondary differential value (for example, see Japanese Patent Application (JP-A) Laid-Open No. 2015-102542.).
PCT publication number WO 2020/105699 A1 describes a diagnostic assistance method for a disease based on an endoscopic image of a digestive organ with use of a convolutional neural network (CNN) according to one embodiment of the present invention trains the CNN using a first endoscopic image of the digestive organ and at least one final diagnosis result of the positivity or the negativity for the disease in the digestive organ, or information corresponding to a severity level, the final diagnosis result being corresponding to the first endoscopic image, and the trained CNN outputs at least one of a probability of the positivity and/or the negativity for the disease in the digestive organ, a severity level of the disease, or a probability corresponding to the invasion depth (infiltration depth) of the disease, based on a second endoscopic image of the digestive organ. According to this embodiment, it is possible to acquire the probability of the positivity and/or the negativity for the disease in the digestive organ, the severity level of the disease, and the invasion depth of the disease, of the subject, at an accuracy substantially comparable to that of an endoscopy specialist within a short time period. and it becomes possible to select a subject requiring a separate confirmation diagnosis within a short time period.
US 2009/0312644 A1 discloses a vital tissue discrimination device having an infrared spectrum acquiring section which acquires, by an endoscope and from in vivo tissue, infrared spectrum information for each picture element of an image picked up under irradiation carried out within a wavelength region of from 900 nm to 1,700 nm, and an operating section which discriminates normal from abnormal of the vital tissue for each picture element using a learning classification type algorithm - the vital tissue identified as abnormal being a tumor of digestive system, illustrated exclusively by a gastric cancer inner wall.

### SUMMARY OF INVENTION

### Technical Problem

As disclosed in JP-A No. 2009-300131 and JP-A No. 2015-102542, there is known a technique of irradiating biological tissue with near infrared light (for example, light having a wavelength around 800 to 2500 nm) and analyzing an image obtained by the irradiation to diagnose the biological tissue. Near-infrared light has many useful features in observation of the inside of a human body. However, because the wavelength region of the near-infrared light is wide, there is a problem that it is difficult to mount, on the endoscope, an imaging device capable of acquiring the characteristics of all bands.

The disclosure was conceived in view of the above circumstances, and an object of the disclosure is to detect the presence or absence of a tumor by using an image captured by irradiating an area in a living body with light of a specific wavelength.

### Solution to Problem

A first aspect of the invention is an image processing device as defined in independent claim 1.

A second aspect of the invention is an image processing program as defined in independent claim 4.

A third aspect of the disclosure is an image processing method as defined in independent claim 5.

A fourth aspect of the disclosure is an endoscope device, including: a light output unit that outputs light having a wavelength of 955 [nm] to 2025 [nm]; and an imaging device that captures an image when an area in a living body is irradiated with light from the light output unit.

### Advantageous Effects of Invention

The disclosure affords the advantageous effect that the presence or absence of a tumor can be detected using an image captured by irradiating an area in a living body with light of a specific wavelength.

### BRIEF DESCRIPTION OF DRAWINGS

Fig. 1 is a diagram illustrating a schematic configuration of an endoscope image processing system according to an embodiment of the disclosure.
Fig. 2 is a diagram to illustrate a GIST.
Fig. 3 is an enlarged view of a distal end of an insertion portion of an endoscope device.
Fig. 4 is a diagram to illustrate an image generated using the present embodiment.
Fig. 5 is a diagram illustrating an example of a functional configuration of an image processing device according to the present embodiment.
Fig. 6 is a diagram illustrating an example of a learned model of the present embodiment.
Fig. 7 is a diagram illustrating a hardware configuration of an image processing device and a control device according to the present embodiment.
Fig. 8 is an example of an image processing routine according to the present embodiment.
Fig. 9 is a diagram to illustrate an Example.
Fig. 10 is a diagram to illustrate an Example.
Fig. 11 is a diagram to illustrate an Example.
Fig. 12 is a diagram to illustrate an Example.
Fig. 13 is a diagram to illustrate an non-claimed Example.
Fig. 14 is a diagram to illustrate an non-claimed Example.
Fig. 15 is a diagram to illustrate an non-claimed Example.
Fig. 16 is a diagram to illustrate an non-claimed Example.
Fig. 17 is a diagram to illustrate an non-claimed Example.
Fig. 18 is a diagram to illustrate an non-claimed Example.
Fig. 19 is a diagram to illustrate an non-claimed Example.
Fig. 20 is a diagram to illustrate an non-claimed Example.
Fig. 21 is a diagram to illustrate an non-claimed Example.
Fig. 22 is a diagram to illustrate an non-claimed Example.
Fig. 23 is a diagram to illustrate an non-claimed Example.
Fig. 24 is a diagram to illustrate an non-claimed Example.
Fig. 25 is a diagram to illustrate an non-claimed Example.
Fig. 26 is a diagram to illustrate an non-claimed Example.
Fig. 27 is a diagram to illustrate an non-claimed Example.

### DESCRIPTION OF EMBODIMENTS

An example of an embodiment of the disclosure will be described hereinbelow with reference to the drawings. Note that, in the drawings, the same or equivalent constituent elements and portions are assigned the same reference signs. Furthermore, the dimensional ratios in the drawings are exaggerated for convenience of description, and are sometimes different from the actual ratios.

### (Configuration of Endoscope Image Processing System 1)

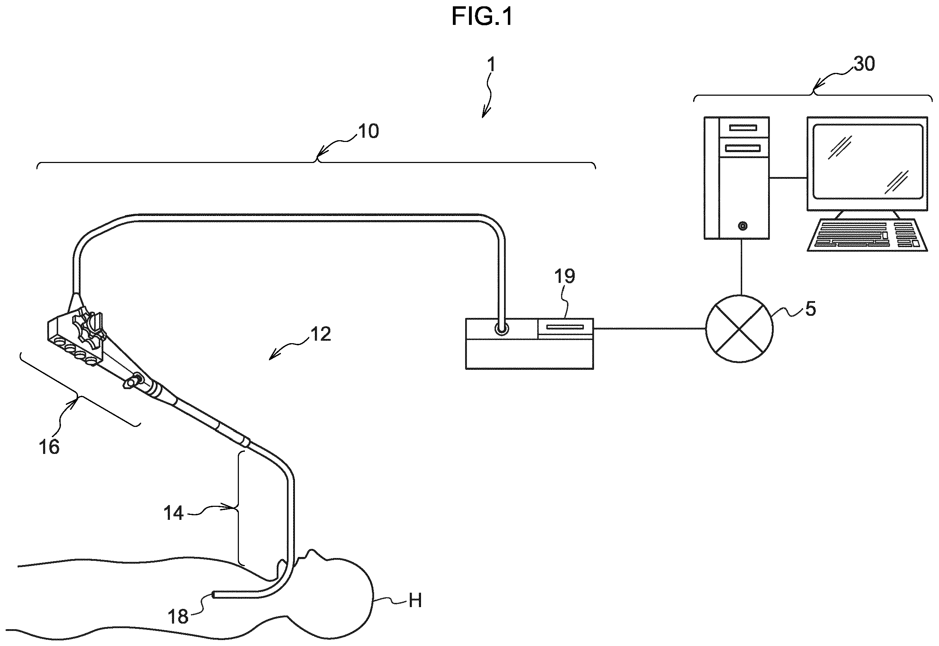

Fig. 1 is a diagram illustrating a schematic configuration of an endoscope image processing system 1 according to an embodiment of the disclosure. As illustrated in Fig. 1, the endoscope image processing system 1 according to the present embodiment includes an endoscope system 10 and an image processing device 30. The endoscope system 10 and the image processing device 30 are connected via a predetermined communication line 5.

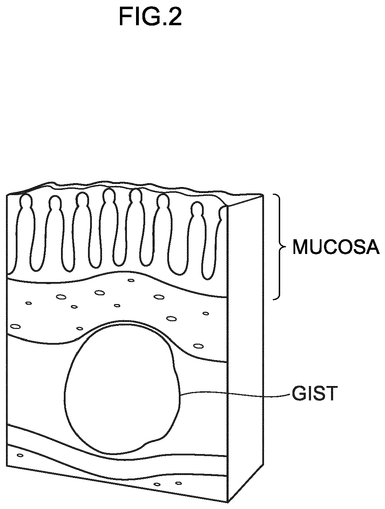

The endoscope image processing system 1 according to the present embodiment detects a gastrointestinal stromal tumor (hereinafter, simply referred to as "GIST".), which is a malignant tumor occurring under the mucosa of the gastrointestinal tract such as the stomach or the small intestine. Fig. 2 is a diagram to illustrate a GIST. As illustrated in Fig. 2, because a GIST occurs under the mucosa of the gastrointestinal tract, it is a tumor which is difficult to detect early.

Conventionally, biological tissue has been irradiated with near-infrared light in all wavelength regions (for example, light having a wavelength around 800 to 2500 nm), and an image of the biological tissue at that time has been captured. In this case, it is necessary to mount, on the endoscope, a camera (for example, a near-infrared hyperspectral camera or the like) capable of capturing images of near-infrared light in all wavelength regions. However, it is difficult to mount such a camera on the endoscope.

Therefore, according to the present embodiment, light of a specific wavelength useful for discriminating the GIST from the near-infrared light is selected. The endoscope image processing system 1 according to the present embodiment irradiates a gastrointestinal tract area with light of a specific wavelength selected in advance, and captures an image of the gastrointestinal tract area at that time. Further, the endoscope image processing system 1 according to the present embodiment discriminates, based on the captured image, the presence or absence of a GIST at the gastrointestinal tract area.

A specific description will be provided hereinbelow.

### (Endoscope System)

As illustrated in Fig. 1, an endoscope system 10 includes an endoscope device 12 and a control device 19. The endoscope device 12 and the control device 19 are electrically connected so as to be able to communicate with each other. The endoscope device 12 photographs the inside of a human body H. The control device 19 generates an image of the inside of the living body of the human body H, based on a signal obtained by imaging.

The endoscope device 12 includes an insertion portion 14 that is inserted into the human body H. The insertion portion 14 is attached to an operation unit 16. The operation unit 16 includes various buttons for instructing an operation such that a distal end 18 of the insertion portion 14 is curved in the vertical direction and the horizontal direction within a predetermined angle range, collecting a tissue sample by operating a puncture needle attached to the distal end 18 of the endoscope device 12, and spraying medicine.

The endoscope device 12 according to the present embodiment is an endoscope for the gastrointestinal tract, and the distal end 18 is inserted into the gastrointestinal tract of the human body H. A light output unit is provided at the distal end 18 of the insertion portion 14 of the endoscope device 12, and light outputted from the light output unit is irradiated onto the gastrointestinal tract area in the living body. Further, the endoscope device 12 acquires an image of the gastrointestinal tract, which is the subject, using an imaging optical system.

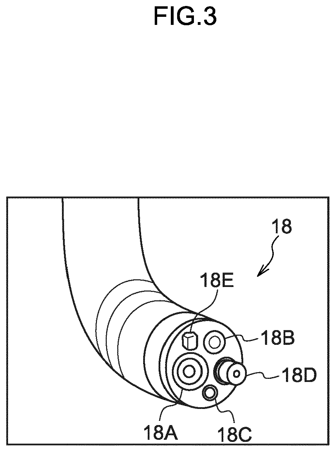

Fig. 3 is an enlarged view of the distal end 18 of the insertion portion 14 of the endoscope device 12. As illustrated in Fig. 3, the distal end 18 of the insertion portion 14 is provided with a camera 18A as an example of an imaging device, and light guides 18B and 18C capable of outputting light of a specific wavelength. The light outputted from the light guides 18B and 18C is light guided by an optical fiber from a light source device (not illustrated) provided to the control device 19. As illustrated in Fig. 3, the distal end 18 of the insertion portion 14 is provided with a forceps opening 18D and a nozzle 18E. Instruments for performing various medical procedures enter and exit from the forceps opening 18D. Moreover, water, air, or the like is outputted from the nozzle 18E.

Light of a specific wavelength is outputted from the light source device of the endoscope device 12 according to the present embodiment, and light of the specific wavelength is outputted from the light guides 18B and 18C, which are examples of the light output unit. Specifically, the light source device is configured to be capable of outputting light having a wavelength of 1000 [nm] to 1500 [nm].

More specifically, the light source device (not illustrated) of the control device 19 is configured to be capable of outputting light having a wavelength of 1050 to 1105 [nm] (hereinafter simply referred to as "first light".), light having a wavelength of 1145 to 1200 [nm] (hereinafter simply referred to as "second light".), light having a wavelength of 1245 to 1260 [nm] (hereinafter simply referred to as "third light".), and light having a wavelength of 1350 to 1405 [nm] (hereinafter simply referred to as "fourth light".). Such light components are light components of a specific wavelength selected in advance.

The endoscope device 12 performs control to irradiate the gastrointestinal tract area in the living body with the first light, and captures an image (hereinafter simply referred to as a "first image".) using the camera 18A at that time. Furthermore, the endoscope device 12 performs control to irradiate the gastrointestinal tract area in the living body with the second light, and captures an image (hereinafter simply referred to as a "second image".) using the camera 18A at that time. The endoscope device 12 performs control to irradiate the gastrointestinal tract area in the living body with the third light, and captures an image (hereinafter simply referred to as a "third image".) using the camera 18A at that time. The endoscope device 12 performs control to irradiate the gastrointestinal tract area in the living body with the fourth light, and captures an image (hereinafter simply referred to as a "fourth image".) using the camera 18A at that time.

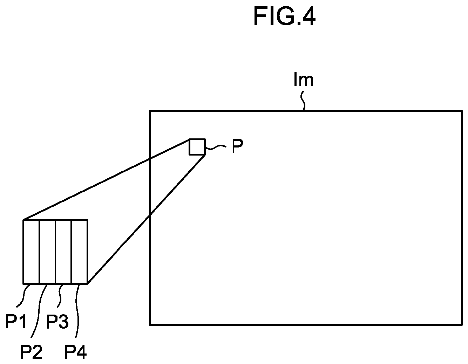

The control device 19 acquires each image captured by the camera of the endoscope device 12. The control device 19 integrates the first image, the second image, the third image, and the fourth image to generate an image Im of the gastrointestinal tract area as illustrated in Fig. 4. As illustrated in Fig. 4, the pixel P of the image Im of the gastrointestinal tract area is obtained by arranging the pixel P1 of the first image, the pixel P2 of the second image, the pixel P3 of the third image, and the pixel P4 of the fourth image in the same position in each of the first image, the second image, the third image, and the fourth image.

The control device 19 then transmits the image Im of the gastrointestinal tract area to the image processing device 30.

### (Image Processing Device)

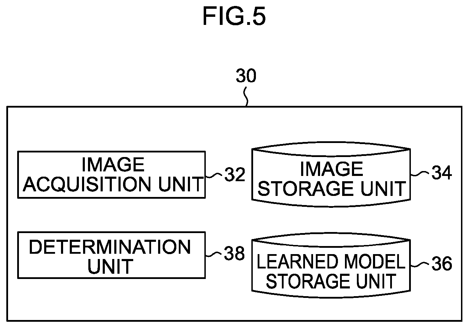

Fig. 5 is a block diagram representing the functional configuration of the image processing device 30. As illustrated in Fig. 5, the image processing device 30 includes an image acquisition unit 32, an image storage unit 34, a learned model storage unit 36, and a determination unit 38.

The image acquisition unit 32 acquires the image Im of the gastrointestinal tract area transmitted from the control device 19. The image acquisition unit 32 then temporarily stores the image Im of the gastrointestinal tract area, in the image storage unit 34.

The image storage unit 34 stores the image Im of the gastrointestinal tract area.

The learned model storage unit 36 stores a learned model generated in advance for detecting a GIST that is present inside the gastrointestinal tract area from the image Im of the gastrointestinal tract area.

The learned model according to the present embodiment is realized by, for example, a known neural network. The learned model according to the present embodiment is a model generated in advance based on data in which an in-vivo image for training and information (so-called label) indicating whether or not a GIST is present inside a gastrointestinal tract area appearing in the in-vivo image are associated with each other.

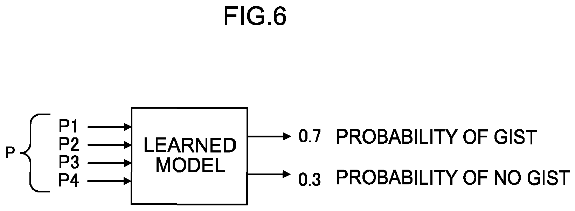

Fig. 6 illustrates an example of a learned model according to the present embodiment. As illustrated in Fig. 6, in the present embodiment, the pixel values P1 to P4 of the pixels P of the image Im of the gastrointestinal tract area are inputted to the learned model. From the learned model, a probability indicating whether or not a GIST is present at a point corresponding to the pixel P is outputted. As illustrated in Fig. 6, for example, a probability 0.7 of being a GIST and a probability 0.3 of not being a GIST are outputted from the learned model. Whether or not the GIST is present is determined for each of a plurality of pixels included in the image Im of the gastrointestinal tract area.

The determination unit 38 inputs the pixel value of each pixel of the image Im of the gastrointestinal tract area stored in the image storage unit 34 to the learned model stored in the learned model storage unit 36, and determines, for each pixel in the image Im of the gastrointestinal tract area, whether or not a GIST is present.

For example, in a case in which a certain pixel is inputted to the learned model, when the probability of being a GIST is higher than the probability of not being a GIST, the determination unit 38 determines that a GIST is present in the point corresponding to the pixel. Furthermore, in a case in which a certain pixel is inputted to the learned model, when the probability of being a GIST is equal to or less than the probability of not being a GIST, the determination unit 38 determines that a GIST is not present at a point corresponding to the pixel.

The determination unit 38 outputs, to a display unit (not illustrated), the determination result regarding the presence or absence of a GIST for each pixel in the image Im of the gastrointestinal tract area.

A display unit (not illustrated) displays the determination result, outputted from the determination unit 38, regarding the presence or absence of a GIST. Note that the determination result regarding the presence or absence of a GIST is outputted, for example, in a format superimposed on the image Im of the gastrointestinal tract area (for example, the point where the GIST is present is displayed in red.). The user then checks the determination result displayed on the display unit.

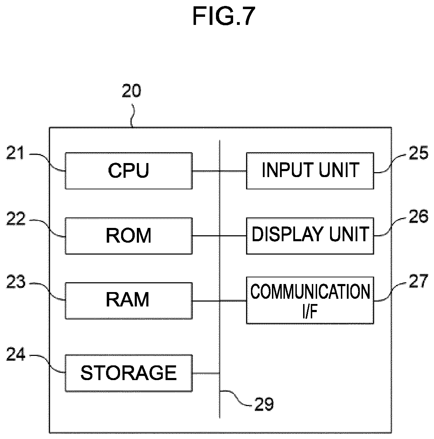

Fig. 7 is a block diagram illustrating a hardware configuration of a computer 20 which is a component of the control device 19 and the image processing device 30. As illustrated in Fig. 7, the computer 20 includes a central processing unit (CPU) 21, a read only memory (ROM) 22, a random access memory (RAM) 23, a storage 24, an input unit 25, a display unit 26, and a communication interface (I/F) 27. The respective configurations are communicably connected to each other via a bus 29.

The CPU 21 is a central processing unit, and executes various programs and controls each unit. That is, the CPU 21 reads a program from the ROM 22 or the storage 24, and executes the program using the RAM 23 as a work area. The CPU 21 performs control of each of the foregoing configurations and various types of arithmetic processing according to the program stored in the ROM 22 or the storage 24. In the present embodiment, the ROM 22 or the storage 24 stores various programs for processing information inputted from the input device.

The ROM 22 stores various programs and various data. Serving as a work area, the RAM 23 temporarily stores programs or data. The storage 24 is configured from a hard disk drive (HDD) or a solid state drive (SSD) or the like, and stores various programs including an operating system, and various data.

The input unit 25 includes a pointing device such as a mouse, and a keyboard, and is used to perform various inputs.

The display unit 26 is, for example, a liquid crystal display, and displays various types of information. The display unit 26 may function as the input unit 25 by adopting a touch panel system.

The communication I/F 27 is an interface for communicating with another device such as an input device, and for example, standards such as Ethernet (registered trademark), FDDI, and Wi-Fi (registered trademark) are used.

Next, the operation of the endoscope image processing system 1 will be described.

When the distal end 18 of the insertion portion 14 of the endoscope device 12 is inserted into the living body in response to operation by the user and the distal end 18 reaches the gastrointestinal tract area, imaging of an image of the gastrointestinal tract area is started.

The endoscope device 12 performs control to irradiate the gastrointestinal tract area with the first light, and captures the first image by using the camera 18A at that time. Further, the endoscope device 12 performs control to irradiate the gastrointestinal tract area with the second light, and captures the second image by using the camera 18A at that time. Further, the endoscope device 12 performs control to irradiate the gastrointestinal tract area with the third light, and captures the third image by using the camera 18A at that time. The endoscope device 12 performs control to irradiate the gastrointestinal tract area with the fourth light, and captures the fourth image by using the camera 18A at that time.

The control device 19 integrates the first image, the second image, the third image, and the fourth image to generate an image Im of the gastrointestinal tract area as illustrated in Fig. 4. The control device 19 then transmits the image Im of the gastrointestinal tract area to the image processing device 30.

When acquiring the image Im of the gastrointestinal tract area transmitted from the control device 19, the image acquisition unit 32 of the image processing device 30 stores the image Im of the gastrointestinal tract area, in the image storage unit 34.

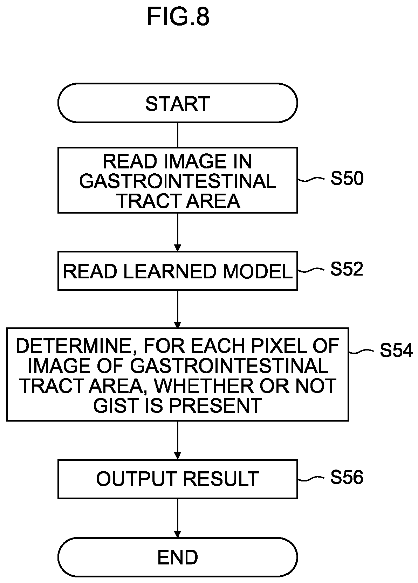

Further, upon receiving an instruction signal to start determination processing to determine whether or not a GIST is present at each point of the image Im of the gastrointestinal tract area, the image processing device 30 executes the image processing routine illustrated in Fig. 8.

Specifically, the CPU 21 performs image processing by reading the image processing program from the ROM 22 or the storage 24, expanding the image processing program in the RAM 23, and executing the image processing program.

In step S50, the image acquisition unit 32 reads the image Im of the gastrointestinal tract area stored in the image storage unit 34.

In step S52, the determination unit 38 reads the learned model stored in the learned model storage unit 36.

In step S54, the determination unit 38 inputs the pixel value of each pixel of the image Im of the gastrointestinal tract area read in step S50 to the learned model read in step S52, and determines whether or not there is a GIST for each pixel in the image Im of the gastrointestinal tract area.

In step S56, the determination unit 38 outputs the determination result regarding the presence or absence of a GIST for each pixel in the image Im of the gastrointestinal tract area to the display unit 26, and terminates the image processing routine.

The display unit 26 displays the determination result regarding the presence or absence of a GIST thus outputted from the determination unit 38.

As described above, the endoscope device according to the present embodiment outputs light having a wavelength of 1000 [nm] to 1500 [nm], more specifically, first light representing light having a wavelength of 1050 to 1105 [nm], second light representing light having a wavelength of 1145 to 1200 [nm], third light representing light having a wavelength of 1245 to 1260 [nm], and fourth light representing light having a wavelength of 1350 to 1405 [nm], and captures an image when the gastrointestinal tract area in the living body is irradiated with the light. Further, the image processing device according to the present embodiment inputs the image of the gastrointestinal tract area to a learned model generated in advance for detecting a GIST that is present inside the gastrointestinal tract area from the image of the gastrointestinal tract area, and determines whether or not a GIST is present at each point of the image. Thus, the presence or absence of a GIST can be detected using an image captured by irradiating a gastrointestinal tract area in a living body with light of a specific wavelength. As a result, for example, the presence or absence of a GIST can be detected without mounting a large-scale imaging device such as a near-infrared hyperspectral camera on an endoscope device.

### [Example]

### (GIST-related Example)

Next, a method of selecting light of a specific wavelength according to the present embodiment will be described as an Example. In the present embodiment, when light having a specific wavelength is selected from near-infrared light, light having a wavelength useful for GIST detection is selected using a neural network which is an example of a learned model obtained by machine learning and using partial least square discriminant analysis (PLS-DA) which is an example of a statistical model obtained by statistical analysis.

Table 1 shows the number of training data used in generating the neural network and the PLS-DA. As described above, in the present embodiment, because the presence or absence of a GIST is determined for each pixel, the number of pieces of training data corresponds to the number of pixels. Note that "Tumor" in Table 1 represents a pixel in which GIST is present, and "Normal" represents a pixel corresponding to a normal region in which a GIST is not present. Furthermore, the number at the left end of Table 1 represents the date on which the image was collected; for example, "20160923 " represents September 23, 2016.

**[Table 1]**

| | Tumor | Normal | All |
|---|---|---|---|
| 20160923 | 27760 | 15522 | 43282 |
| 20160930 | 6090 | 5382 | 11472 |
| 20171115 | 18619 | 1917 | 20536 |
| 20171215 | 6399 | 12936 | 19335 |
| 20180202 | 9617 | 2332 | 11949 |
| All | 68485 | 38089 | 106574 |

### (Wavelength Selection Using Neural Network)

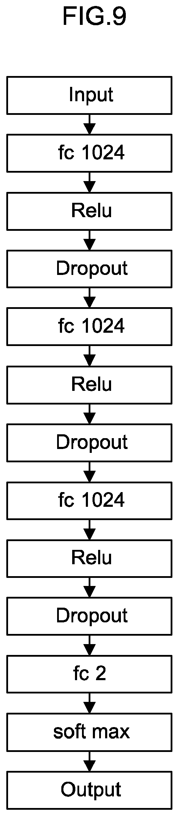

Fig. 9 illustrates a configuration of a neural network used for wavelength selection in this Example. In Fig. 9, "Input" represents an input layer, and "Output" represents an output layer. Further, "fc" in Fig. 9 represents a fully connected layer, and the number written together represents the number of units. Further, "Relu" represents a rectified linear unit (ReLU) which is a known activation function. In addition, "Dropout " indicates that dropout, which is a known method, is used. Further, "softmax " represents a known softmax function.

In the present embodiment, the neural network of Fig. 9 was learned using the training data of Table 1. The contribution amount of light of each wavelength was then calculated using the learned weight parameter of the neural network. The contribution amount refers to which wavelength of light has a strong influence on correctly identifying the input, and is calculated based on a result of performing forward propagation calculation on an input in which all inputs of wavelengths other than a certain wavelength are set to 0. The detailed steps to calculate the contribution amount are as follows.
(1) A learned neural network is generated using the training data set. One piece of training data is data in which a pixel value of a certain pixel in an image captured when a gastrointestinal tract area is irradiated with light of each wavelength is associated with a label indicating whether the pixel is a GIST. Note that, when the neural network is learned, the weights of only the fully connected layers in the neural network are learned. Further, the neural network has no bias, and the activation function is the Relu function.
(2) One piece of data is selected from the training data set, and a pixel value for each wavelength of the data is inputted to the learned neural network to calculate forward propagation. At this time, the output values of all the nodes in the learned neural network are recorded. Note that the output value refers to both a value outputted by each node of the neural network and a value outputted by the output layer of the neural network.
(3) The pixel value corresponding to one wavelength is selected from the pixel values for each wavelength in the one piece of data used in (2) above, and the pseudo forward propagation is calculated using the learned neural network with the values of the pixel values of the other wavelengths set to 0. The output of the learned neural network at this time is set as a contribution amount of one wavelength of one piece of data. Note that, at this time, the Relu function of the learned neural network is not applied. At this time, for each node of the learned neural network, the output value of the node for which the value recorded in (2) above is 0 or less is calculated as 0.
(4) (3) above is repeated for all wavelengths of one piece of data, and the contribution amount for the input of the pixel value corresponding to each wavelength is obtained.
(5) (2) to (4) above are repeated for a plurality of pieces of training data to obtain data of the contribution amounts for the plurality of pieces of training data.

The contribution amount will be described in specific terms hereinbelow.

Two values which are ultimately outputted by the learned neural network are, for example, {0.7, 0.3} (indicating a tumor or a normal pixel). The larger of the two output values of the learned neural network is selected, and it is specified whether the pixel is a tumor pixel or a normal pixel. For example, in a case in which the output value when the pixel value of a certain pixel is inputted to the learned neural network is {0.7, 0.3}, the pixel is determined to be a tumor.

When the pixel values of the pixels corresponding to all the wavelengths have been inputted to the learned neural network, there exist, among those wavelengths, wavelengths that contribute to a correct output and wavelengths that contribute to an incorrect output. For example, consider a case in which correct data of a certain pixel is {1, 0} and the pixel is a tumor. In this case, it is assumed that {0.7, 0.3} is outputted in a case in which the pixel values corresponding to all the wavelengths of the pixels have been inputted to the learned neural network. A wavelength that directs this output value toward {1, 0} is a wavelength that contributes to the correct output, and a wavelength that directs this output value toward {0, 1} is a wavelength that contributes to the incorrect output.

Therefore, one piece of data (the pixel value of one pixel) of the training data is inputted to the learned neural network with a value other than a certain wavelength as 0, and a final output value is calculated. In this case, the output of the node that directs the final output value in the wrong direction is set to 0. This processing corresponds to (2) and (3) above.

Further, for a certain wavelength, the sum of the output values calculated as described above for a plurality of pieces of data (the pixel values of a plurality of pixels) is calculated and set as the contribution amount of the wavelength. In this case, in a case in which the correct data of a certain pixel is {1, 0} and a pixel value corresponding to a certain wavelength is inputted to the learned neural network as {0.8, 0.2}, 0.8 is the calculated value. The calculated value thus calculated is also calculated for a plurality of pixels, and the sum of the calculated values calculated for the plurality of pixels is set as the contribution amount. The same calculation is executed for each of the plurality of wavelengths, and the contribution amounts of the wavelengths are calculated.

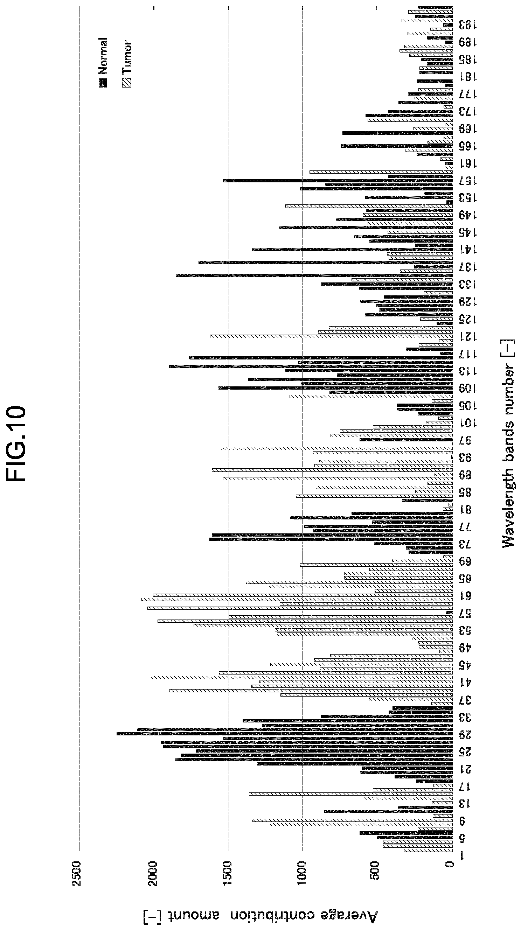

Fig. 10 illustrates contribution amounts obtained through calculation. The vertical axis in Fig. 10 represents the contribution amount (denoted as the "Average contribution amount" in the drawing), and the horizontal axis represents the wavelength (denoted as the "Wavelength bands number" in the drawing). Note that each number of the wavelength on the horizontal axis represents each wavelength, the number "1" corresponds to a wavelength of 913.78 [nm], and the number "193" corresponds to a wavelength of 2126.27 [nm]. Note that the wavelength increases by 6.28 to 6.34 [nm] every time the number increases by 1. Tables 2 and 3 below show correspondence relationships between numbers and wavelengths.

**[Table 2]**

| Wavelength number | Wavelength [nm] | Wavelength number | Wavelength [nm] | Wavelength number | Wavelength [nm] | Wavelength number | Wavelength [nm] |
|---|---|---|---|---|---|---|---|
| 1 | 913.78 | 33 | 1116.4 | 65 | 1318.8 | 97 | 1520.99 |
| 2 | 920.11 | 34 | 1122.72 | 66 | 1325.12 | 98 | 1527.3 |
| 3 | 926.45 | 35 | 1129.05 | 67 | 1331.44 | 99 | 1533.62 |
| 4 | 932.78 | 36 | 1135.38 | 68 | 1337.76 | 100 | 1539.93 |
| 5 | 939.12 | 37 | 1141.71 | 69 | 1344.08 | 101 | 1546.25 |
| 6 | 945.45 | 38 | 1148.03 | 70 | 1350.4 | 102 | 1552.56 |
| 7 | 951.78 | 39 | 1154.36 | 71 | 1356.72 | 103 | 1558.87 |
| 8 | 958.12 | 40 | 1160.69 | 72 | 1363.05 | 104 | 1565.19 |
| 9 | 964.45 | 41 | 1167.02 | 73 | 1369.37 | 105 | 1571.5 |
| 10 | 970.79 | 42 | 1173.34 | 74 | 1375.69 | 106 | 1577.81 |
| 11 | 977.12 | 43 | 1179.67 | 75 | 1382.01 | 107 | 1584.13 |
| 12 | 983.45 | 44 | 1186 | 76 | 1388.32 | 108 | 1590.44 |
| 13 | 989.78 | 45 | 1192.32 | 77 | 1394.64 | 109 | 1596.75 |
| 14 | 996.12 | 46 | 1198.65 | 78 | 1400.96 | 110 | 1603.06 |
| 15 | 1002.45 | 47 | 1204.97 | 79 | 1407.28 | 111 | 1609.38 |
| 16 | 1008.78 | 48 | 1211.3 | 80 | 1413.6 | 112 | 1615.69 |
| 17 | 1015.11 | 49 | 1217.62 | 81 | 1419.92 | 113 | 1622 |
| 18 | 1021.44 | 50 | 1223.95 | 82 | 1426.24 | 114 | 1628.31 |
| 19 | 1027.78 | 51 | 1230.27 | 83 | 1432.56 | 115 | 1634.62 |
| 20 | 1034.11 | 52 | 1236.6 | 84 | 1438.87 | 116 | 1640.94 |
| 21 | 1040.44 | 53 | 1242.92 | 85 | 1445.19 | 117 | 1647.25 |
| 22 | 1046.77 | 54 | 1249.25 | 86 | 1451.51 | 118 | 1653.56 |
| 23 | 1053.1 | 55 | 1255.57 | 87 | 1457.83 | 119 | 1659.87 |
| 24 | 1059.43 | 56 | 1261.89 | 88 | 1464.14 | 120 | 1666.18 |
| 25 | 1065.76 | 57 | 1268.22 | 89 | 1470.46 | 121 | 1672.49 |
| 26 | 1072.09 | 58 | 1274.54 | 90 | 1476.78 | 122 | 1678.8 |
| 27 | 1078.42 | 59 | 1280.86 | 91 | 1483.09 | 123 | 1685.11 |
| 28 | 1084.75 | 60 | 1287.19 | 92 | 1489.41 | 124 | 1691.42 |
| 29 | 1091.08 | 61 | 1293.51 | 93 | 1495.73 | 125 | 1697.73 |
| 30 | 1097.41 | 62 | 1299.83 | 94 | 1502.04 | 126 | 1704.04 |
| 31 | 1103.74 | 63 | 1306.15 | 95 | 1508.36 | 127 | 1710.34 |
| 32 | 1110.07 | 64 | 1312.48 | 96 | 1514.67 | 128 | 1716.65 |

**[Table 3]**

| Wavelength number | Wavelength [nm] | Wavelength number | Wavelength [nm] | Wavelength number | Wavelength [nm] | Wavelength number | Wavelength [nm] |
|---|---|---|---|---|---|---|---|
| 129 | 1722.96 | 161 | 1924.72 | 193 | 2126.27 | 225 | 2327.6 |
| 130 | 1729.27 | 162 | 1931.02 | 194 | 2132.56 | 226 | 2333.89 |
| 131 | 1735.58 | 163 | 1937.32 | 195 | 2138.86 | 227 | 2340.18 |
| 132 | 1741.89 | 164 | 1943.63 | 196 | 2145.15 | 228 | 2346.46 |
| 133 | 1748.19 | 165 | 1949.93 | 197 | 2151.45 | 229 | 2352.75 |
| 134 | 1754.5 | 166 | 1956.23 | 198 | 2157.74 | 230 | 2359.04 |
| 135 | 1760.81 | 167 | 1962.53 | 199 | 2164.03 | 231 | 2365.33 |
| 136 | 1767.11 | 168 | 1968.83 | 200 | 2170.33 | 232 | 2371.61 |
| 137 | 1773.42 | 169 | 1975.13 | 201 | 2176.62 | 233 | 2377.9 |
| 138 | 1779.73 | 170 | 1981.43 | *202* | 2182.91 | 234 | 2384.19 |
| 139 | 1786.03 | 171 | 1987.73 | 203 | 2189.21 | 235 | 2390.47 |
| 140 | 1792.34 | 172 | 1994.03 | 204 | 2195.5 | 236 | 2396.76 |
| 141 | 1798.65 | 173 | 2000.33 | 205 | 2201.79 | 237 | 2403.04 |
| 142 | 1804.95 | 174 | 2006.63 | 206 | 2208.08 | 238 | 2409.33 |
| 143 | 1811.26 | 175 | 2012.92 | 207 | 2214.38 | 239 | 2415.62 |
| 144 | 1817.56 | 176 | 2019.22 | 208 | 2220.67 | 240 | 2421.9 |
| 145 | 1823.87 | 177 | 2025.52 | 209 | 2226.96 | 241 | 2428.19 |
| 146 | 1830.17 | 178 | 2031.82 | 210 | 2233.25 | 242 | 2434.47 |
| 147 | 1836.48 | 179 | 2038.12 | 211 | 2239.54 | 243 | 2440.75 |
| 148 | 1842.78 | 180 | 2044.42 | 212 | 2245.83 | 244 | 2447.04 |
| 149 | 1849.09 | 181 | 2050.71 | 213 | 2252.13 | 245 | 2453.32 |
| 150 | 1855.39 | 182 | 2057.01 | 214 | 2258.42 | 246 | 2459.61 |
| 151 | 1861.69 | 183 | 2063.31 | 215 | 2264.71 | 247 | 2465.89 |
| 152 | 1868 | 184 | 2069.6 | 216 | 2271 | 248 | 2472.17 |
| 153 | 1874.3 | 185 | 2075.9 | 217 | 2277.29 | 249 | 2478.46 |
| 154 | 1880.61 | 186 | 2082.2 | 218 | 2283.58 | 250 | 2484.74 |
| 155 | 1886.91 | 187 | 2088.49 | 219 | 2289.87 | 251 | 2491.02 |
| 156 | 1893.21 | 188 | 2094.79 | 220 | 2296.16 | 252 | 2497.31 |
| 157 | 1899.51 | 189 | 2101.09 | 221 | 2302.45 | 253 | 2503.59 |
| 158 | 1905.82 | 190 | 2107.38 | 222 | 2308.73 | 254 | 2509.87 |
| 159 | 1912.12 | 191 | 2113.68 | 223 | 2315.02 | 255 | 2516.15 |
| 160 | 1918.42 | 192 | 2119.97 | 224 | 2321.31 | 256 | 2522.44 |

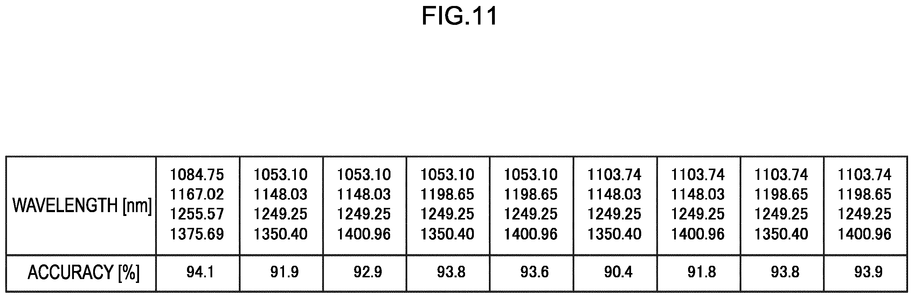

The larger the absolute value of the contribution amounts illustrated in Fig. 10, the more useful the wavelength is for detecting a GIST. In the Example, four wavelengths in a region having a high contribution amount were selected from Fig. 10. Specifically, based on the premise that each wavelength is selected from the peaks having a high contribution amount, the accuracy was as illustrated in Fig. 11.

Accordingly, it may be said that light having a wavelength of 1050 to 1105 [nm], light having a wavelength of 1145 to 1200 [nm], light having a wavelength of 1245 to 1260 [nm], and light having a wavelength of 1350 to 1405 [nm] are light having wavelengths useful for detecting a GIST.

### (Wavelength Selection by PLS-DA)

In the present embodiment, wavelength selection and discrimination are performed using PLS-DA, which is a known statistical method. Specifically, the identification model was generated by PLS-DA by using the training data in Table 1. The contribution amount of the light of each wavelength was calculated using the sum of the factor loads of the light of each wavelength up to the eighth principal component of the generated identification model. Therefore, the contribution amount is the sum of the factor loads of the light of each wavelength up to the eighth principal component of the generated identification model.

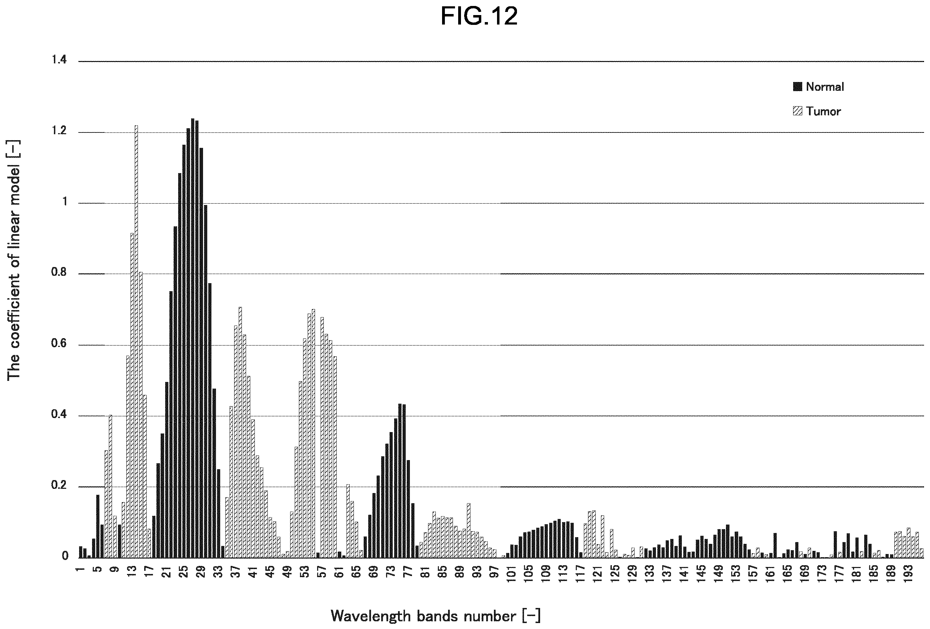

Fig. 12 illustrates contribution amounts obtained through calculation. The vertical axis in Fig. 12 represents the contribution amount (denoted as "The coefficient of linear model" in the drawing). Similarly to the wavelength selection using the neural network, peaks having a high contribution amount are formed, and it is considered that a GIST can be accurately detected by selecting the wavelength from these peaks.

Table 4 below shows the GIST discrimination accuracy using the neural network (denoted as "proposed technique" in Table 4) and the GIST discrimination accuracy by PLS-DA. Note that the number in parentheses in Table 4 represents the number of wavelengths. "After dimension reduction" indicates that four wavelengths are selected from all wavelengths and that the dimension is reduced. As shown in Table 4, detection accuracy substantially equivalent to that in a case in which all the wavelengths are used is obtained only by using light of four selected wavelengths.

**[Table 4]**

| | Proposed method | PLS-DA |
|---|---|---|
| All wavelengths | 97.6% (196) | 93.1% (196) |
| After dimension reduction | 95.9% (4) | 91.4% (4) |

| | | |
|---|---|---|
| Accuracy[%] (Number of wavelengths) | | |

As described above, in a case in which, according to the present embodiment, a GIST is detected using light having a selected wavelength, it can be said that the accuracy in this case is substantially equivalent to the detection accuracy in a case in which the light having the wavelength in all the regions of near-infrared light is used.

### (Non-claimed Lung Cancer-related Example)

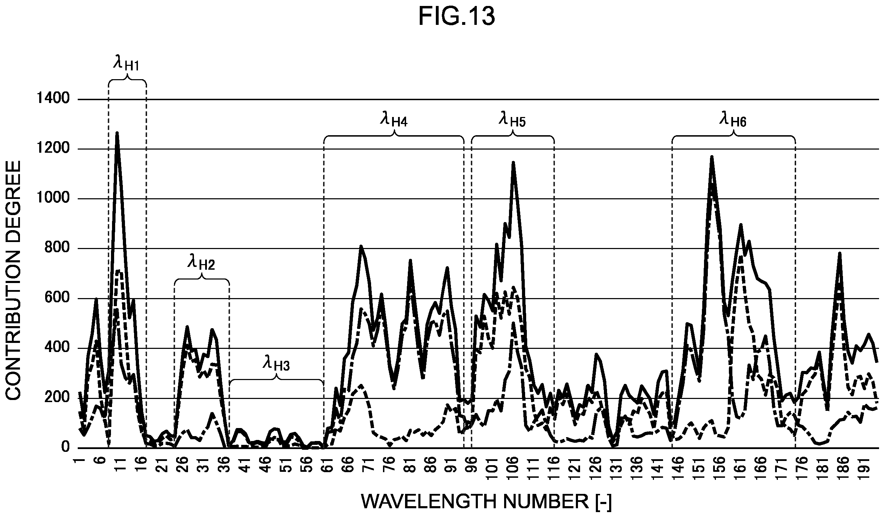

Next, a lung cancer-related Example will be described. Light of a specific wavelength useful for detecting lung cancer was selected using the same method as in the above GIST-related Example. The wavelength range is 196 wavelengths of 913.78 [nm] to 2145.15 [nm]. Fig. 13 illustrates contribution amounts obtained through calculation. The vertical axis in Fig. 13 represents the contribution amount (denoted as "contribution degree" in the drawing), and the horizontal axis represents the wavelength number. Note that each number of the wavelength on the horizontal axis represents each wavelength, the number "1" corresponds to a wavelength of 913.78 [nm], and the number "193" corresponds to a wavelength of 2126.27 [nm].

The larger the absolute value of the contribution amounts illustrated in Fig. 13, the more useful the wavelength is for detecting lung cancer. The broken lines in Fig. 13 represent contribution amounts when a tumor is discriminated as a tumor, a dot-dash line represents the contribution amount when a normal region is discriminated as a normal region, and a solid line represents the sum of these contribution amounts.

In this Example, six wavelengths in a region having a high contribution amount were selected from Fig. 13. Specifically, each wavelength was selected from a peak having a large contribution amount: 977.12 [nm] light was selected from the wavelength range λ_{H1}, 1103.74 [nm] light was selected from the wavelength range λ_{H2}, 1198.65 [nm] light was selected from the wavelength range λ_{H3}, 1350.40 [nm] light was selected from the wavelength range λ_{H4}, 1584.13 [nm] light was selected from the wavelength range λ_{H5}, and 1893.21 [nm] light was selected from the wavelength range λ_{H6}. Although the contribution amount corresponding to the wavelength range λ_{H3} in Fig. 13 is smaller than the contribution amounts of other wavelength ranges, light of 1198.65 [nm] in the wavelength range λ_{H3} was selected because light of the wavelength range λ_{H3} was considered to be useful for identification of lung cancer through trial and error in wavelength selection. Note that the wavelength range λ_{H1} is, for example, a wavelength of 955 to 1020 [nm], the wavelength range λ_{H2} is, for example, a wavelength of 1055 to 1135 [nm], the wavelength range λ_{H3} is, for example, a wavelength of 1135 to 1295 [nm], the wavelength range λ_{H4} is, for example, a wavelength of 1295 to 1510 [nm], the wavelength range λ_{H5} is, for example, a wavelength of 1510 to 1645 [nm], and the wavelength range λ_{H6} is, for example, a wavelength of 1820 to 2020 [nm]. Light having a wavelength of 955 to 1020 [nm] is an example of first light, light having a wavelength of 1055 to 1135 [nm] is an example of second light, light having a wavelength of 1135 to 1295 [nm] is an example of third light, light having a wavelength of 1295 to 1510 [nm] is an example of fourth light, light having a wavelength of 1510 to 1645 [nm] is an example of fifth light, and light having a wavelength of 1820 to 2020 [nm] is an example of sixth light. When the presence or absence of a tumor present in lungs in a living body was detected using an image captured by irradiating the lungs with light of these specific wavelengths, the accuracy was as shown in the following table.

**[Table 5]**

| | Validation Accuracy |
|---|---|
| All wavelengths (196 wavelengths) | 94.3% |
| After dimension reduction (6 wavelengths) | 89.3% (6) |

As shown in the above table, it can be seen that even if the total wavelength (196 wavelengths) is reduced to 6 wavelengths, the accuracy is reduced only by about 5%. Accordingly, it may be said that at least one of light having a wavelength of 955 to 1020 [nm], light having a wavelength of 1055 to 1135 [nm], light having a wavelength of 1135 to 1295 [nm], light having a wavelength of 1295 to 1510 [nm], light having a wavelength of 1510 to 1645 [nm], and light having a wavelength of 1820 to 2020 [nm] is light having a wavelength useful for detecting lung cancer.

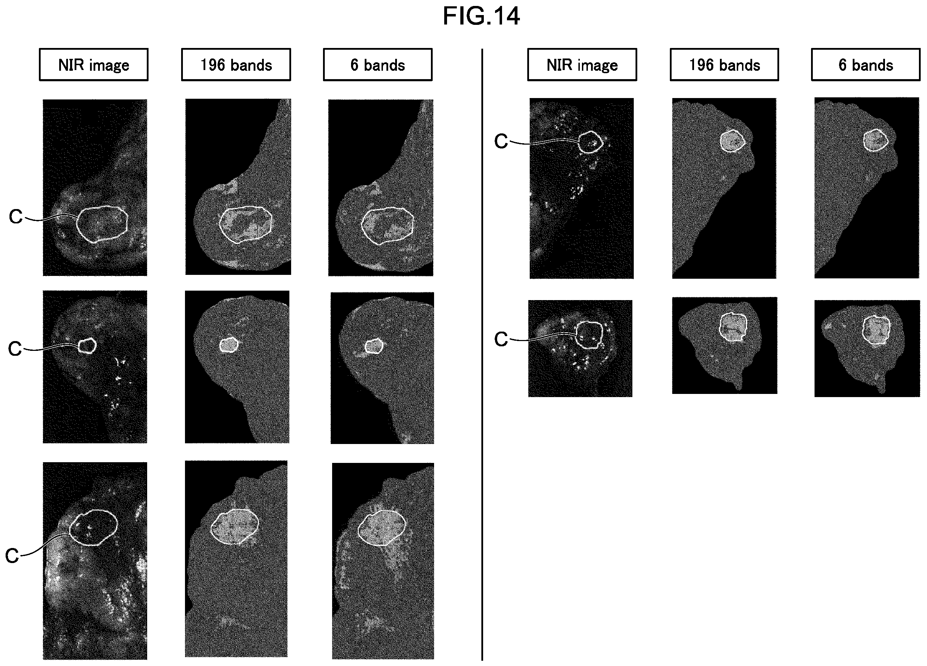

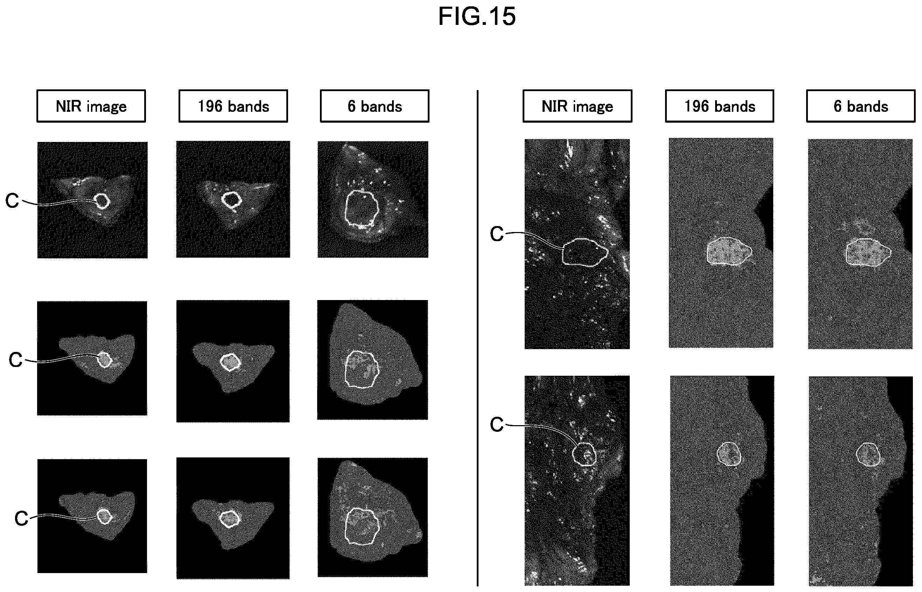

Furthermore, Figs. 14 and 15 illustrate lung cancer identification results. In the identification results shown in Figs. 14 and 15, a tumor C is present in an image of a lung specimen (denoted as "NIR image" in the drawing). In contrast, it can be seen that the identification results are hardly different between the result of discriminating the tumor C based on the image obtained by irradiating the lungs with light of all wavelengths (196 wavelengths) (denoted as "196band" in the drawing) and the result of discriminating the tumor C based on the image obtained by irradiating the lungs with light of six wavelengths selected in this Example (denoted as "6band" in the drawing). Accordingly, it may also be said that at least one of light having a wavelength of 955 to 1020 [nm], light having a wavelength of 1055 to 1135 [nm], light having a wavelength of 1135 to 1295 [nm], light having a wavelength of 1295 to 1510 [nm], light having a wavelength of 1510 to 1645 [nm], and light having a wavelength of 1820 to 2020 [nm] is light having a wavelength useful for detecting lung cancer.

### (Non-claimed Gastric Cancer-related Example)

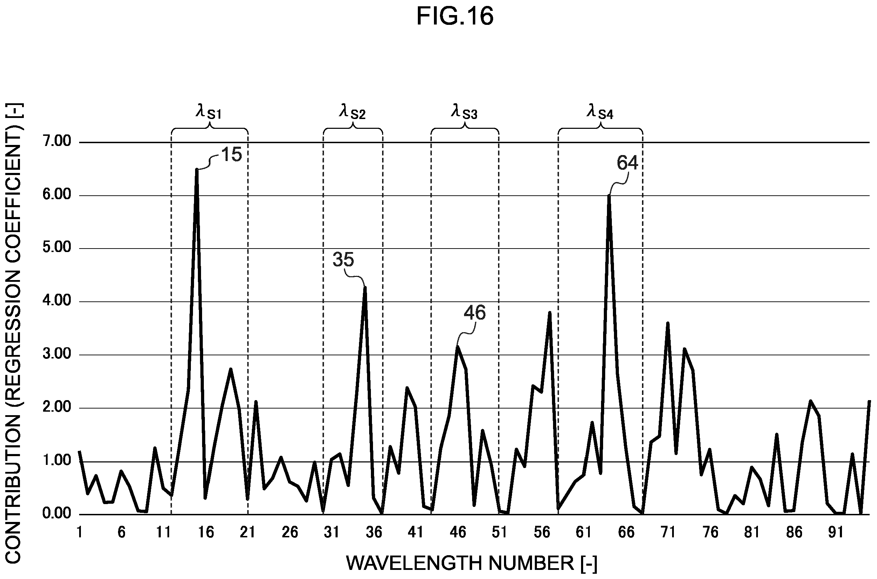

Next, a gastric cancer-related Example will be described. A learned SVM model was generated by replacing the neural network according to the GIST-related embodiment above with a support vector machine (SVM), and light of a specific wavelength useful for detection of gastric cancer was selected by a similar method using LASSO (Least Absolute Shrinkage and Selection Operator) for wavelength selection. When the learned SVM model was generated, learning data of 6 specimens (normal: 405,525 pix, tumor: 107,078 pix) was used. In addition, leave-one-out cross-validation was used for evaluation of the learned SVM. Fig. 16 illustrates the contribution (regression coefficient) of the model obtained by LASSO. In Fig. 16, the vertical axis represents a LASSO regression coefficient, and the horizontal axis represents a wavelength number. Note that each number of the wavelength on the horizontal axis represents each wavelength, the number "1" corresponds to a wavelength of 1002.45 [nm], and the number "91" corresponds to a wavelength of 1571.5 [nm]. Therefore, in the gastric cancer-related Example, the relationship between the wavelength number and the actual wavelength is not as shown in Tables 2 and 3 above, and the number "1" corresponds to a wavelength of 1002.45 [nm] and the number "91" corresponds to a wavelength of 1571.5 [nm].

In this Example, four wavelengths in a region having a high contribution were selected from Fig. 16. Specifically, each wavelength is selected from a peak having a large contribution amount: 1091.08 [nm] light (wavelength number 15) is selected from the wavelength range λ_{S1}, 1217.52 [nm] light (wavelength number 35) is selected from the wavelength range λ_{S2}, 1287.19 [nm] light (wavelength number 46) is selected from the wavelength range λ_{S3}, and 1400.96 [nm] light (wavelength number 64) is selected from the wavelength range λ_{S4}. Note that, when selecting the wavelength, trial and error was performed to select light considered to be useful for identification of gastric cancer. Note that the wavelength range λ_{S1} is, for example, a wavelength of 1065 to 1135 [nm], the wavelength range λ_{S2} is, for example, a wavelength of 1180 to 1230 [nm], the wavelength range λ_{S3} is, for example, a wavelength of 1255 to 1325 [nm], and the wavelength range λ_{S4} is, for example, a wavelength of 1350 to 1425 [nm]. Light having a wavelength of 1065 to 1135 [nm] is an example of first light, light having a wavelength of 1180 to 1230 [nm] is an example of second light, light having a wavelength of 1255 to 1325 [nm] is an example of third light, and light having a wavelength of 1350 to 1425 [nm] is an example of fourth light. When the presence or absence of a tumor present in a stomach in a living body was detected using an image captured by irradiating the stomach with light of these specific wavelengths, the accuracy was as shown in the following table. Note that all the wavelengths (95) correspond to wavelengths from the number "1" wavelength 1002.45 [nm] to the number "95" wavelength 1596.75 [nm].

**[Table 6]**

| | All wavelengths (95) | Selected wavelengths (4) |
|---|---|---|
| Accuracy | 0.784 | 0.800 |
| Precision | 0.920 | 0.773 |
| Recall | 0.746 | 0.968 |
| Specificity | 0.500 | 0.512 |
| F-measure | 0.971 | 0.859 |

As shown in the above table, it can be seen that there is no significant difference in accuracy, precision, recall, specificity, and F-measure between all (95) wavelengths and the selected (4) wavelengths. Accordingly, it may be said that at least one of light having a wavelength of 1065 to 1135 [nm], light having a wavelength of 1180 to 1230 [nm], light having a wavelength of 1255 to 1325 [nm], and light having a wavelength of 1350 to 1425 [nm] is light having a wavelength useful for detecting gastric cancer.

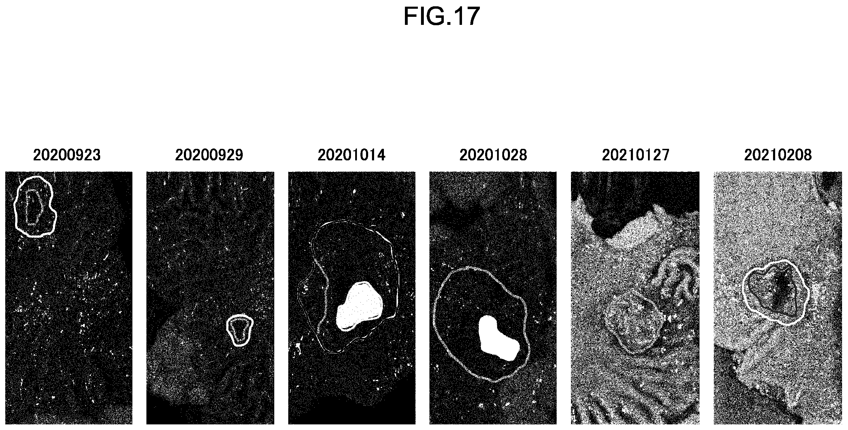

Furthermore, Figs. 17 to 20 illustrate gastric cancer identification results. Fig. 17 are images of a specimen. The numbers in the drawing represent the dates on which the images were taken; for example, "20200923" represents September 23, 2020. The region surrounded by a white line in Fig. 17 is a region corresponding to unexposed tumor tissue; the region surrounded by a gray line is a region corresponding to exposed tumor tissue; and the region filled in white is a region corresponding to tumor tissue in a necrotic state.

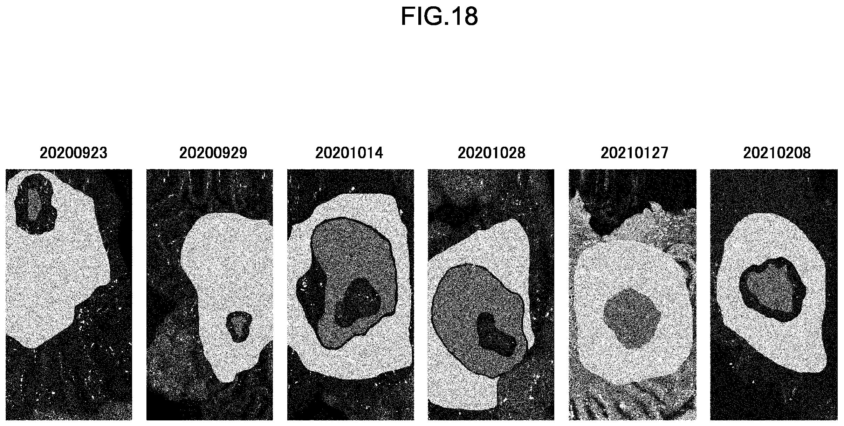

Fig. 18 is an image of training data. The white region in Fig. 18 is a normal region. The gray region in Fig. 18 is a region corresponding to the exposed tumor tissue. Note that, in Fig. 18, tumor tissue in a necrotic state and unexposed tumor tissue are excluded and not displayed.

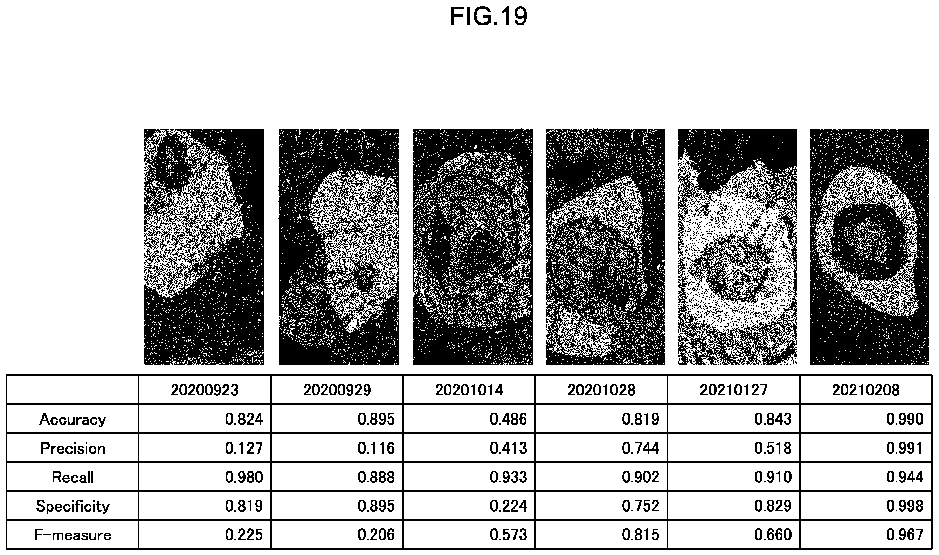

Fig. 19 is a diagram illustrating a region of a tumor estimated from an image obtained by irradiating the stomach with light having a wavelength of 95. A dark gray region is a region which is determined to be a tumor, and a light gray area is a region which is determined to be normal. In addition, the table illustrated in the drawing represents the accuracy, precision, recall, specificity, and F-measure of the determination results for the pixels in each image.

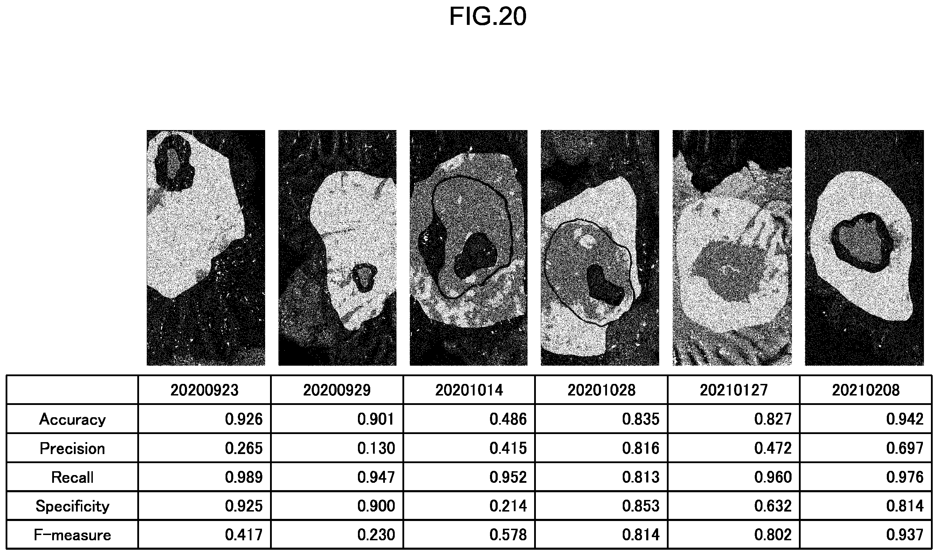

Fig. 20 is a diagram illustrating a region of a tumor estimated from an image obtained by irradiating the stomach with light of four selected wavelengths. A dark gray region is a region which is determined to be a tumor, and a light gray area is a region which is determined to be normal. The table illustrated in the drawing represents the accuracy, precision, recall, specificity, and F-measure of the determination results for the pixels in each image.

When Fig. 19 and Fig. 20 are compared, it can be seen that there is not so much difference in the determination result. Accordingly, it may also be said that at least one of light having a wavelength of 1065 to 1135 [nm], light having a wavelength of 1180 to 1230 [nm], light having a wavelength of 1255 to 1325 [nm], and light having a wavelength of 1350 to 1425 [nm] is light having a wavelength useful for detecting gastric cancer.

### (Non-claimed Tumor-bearing Mouse-related Example)

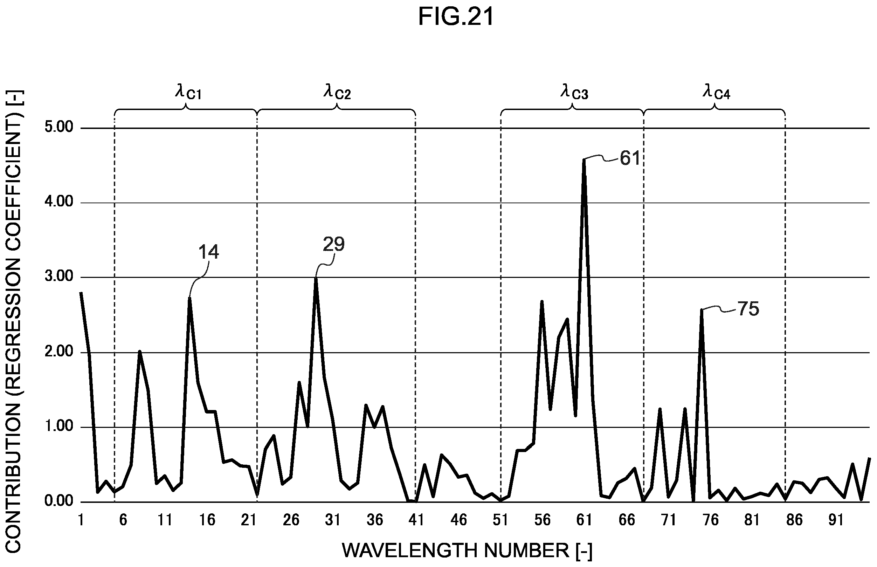

Next, a tumor-bearing mouse-related Example will be described. Light of a specific wavelength useful for detecting a tumor was selected using the same method as the method in the above gastric cancer-related Example. Note that the tumor-bearing mouse according to this Example is a tumor-bearing mouse in which cells derived from human colorectal cancer are used. In this Example, learning data of 11 specimens (normal: 245,866 pix; tumor: 107,078 pix) was used. In addition, leave-one-out cross-validation was used for evaluation of the learned SVM. Fig. 21 illustrates the contribution (regression coefficient) of the model obtained by LASSO. Note that, as per the gastric cancer-related Example, each number of the wavelengths on the horizontal axis represents each wavelength, the number "1" corresponds to a wavelength of 1002.45 [nm], and the number "91" corresponds to a wavelength of 1571.5 [nm]. Therefore, in the tumor-bearing mouse-related Example, the relationship between the wavelength number and the actual wavelength is not as shown in Tables 2 and 3 above, and the number "1" corresponds to a wavelength of 1002.45 [nm] and the number "91" corresponds to a wavelength of 1571.5 [nm].

In this Example, four wavelengths in a region having a high contribution were selected from Fig. 21. Specifically, each wavelength is selected from a peak having a large contribution amount: 1084.75 [nm] light (wavelength number 14) is selected from the wavelength range λ_{C1}, 1179.67 [nm] light (wavelength number 29) is selected from the wavelength range λ_{C2}, 1382.01 [nm] light (wavelength number 61) is selected from the wavelength range λ_{C3}, and 1470.46 [nm] light (wavelength number 75) is selected from the wavelength range λ_{C4}. Note that, when selecting the wavelength, trial and error was performed to select light considered to be useful for identification of a tumor. Note that the wavelength range λ_{C1} is, for example, a wavelength of 1020 to 1140 [nm], the wavelength range λ_{C2} is, for example, a wavelength of 1140 to 1260 [nm], the wavelength range λ_{C3} is, for example, a wavelength of 1315 to 1430 [nm], and the wavelength range λ_{C4} is, for example, a wavelength of 1430 to 1535 [nm]. Light having a wavelength of 1020 to 1140 [nm] is an example of first light, light having a wavelength of 1140 to 1260 [nm] is an example of second light, light having a wavelength of 1315 to 1430 [nm] is an example of third light, and light having a wavelength of 1430 to 1535 [nm] is an example of fourth light. Note that, when selecting the wavelength, trial and error was performed to select light considered to be useful for identification of a mouse-borne tumor. When the presence or absence of a tumor was detected using an image captured by irradiating a tumor-bearing mouse with light of these specific wavelengths, the accuracy was as shown in the following table.

**[Table 7]**

| | All wavelengths (95) | Selected wavelengths (4) |
|---|---|---|
| Accuracy | 0.885 | 0.891 |
| Precision | 0.276 | 0.287 |
| Recall | 0.917 | 0.909 |
| Specificity | 0.883 | 0.890 |
| F-measure | 0.424 | 0.436 |

As shown in the above table, it can be seen that there is no significant difference in accuracy, precision, recall, specificity, and F-measure between all (95) wavelengths and the selected (4) wavelengths.

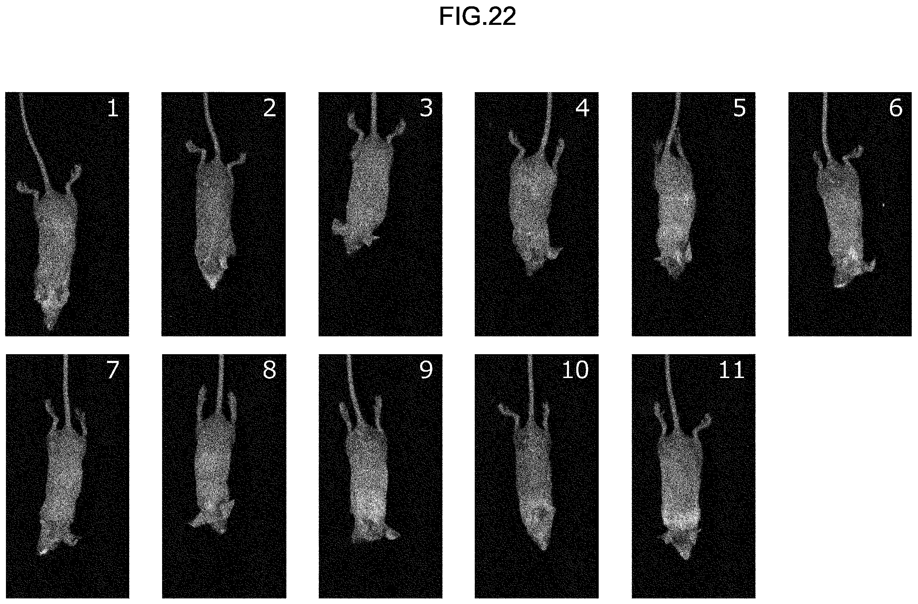

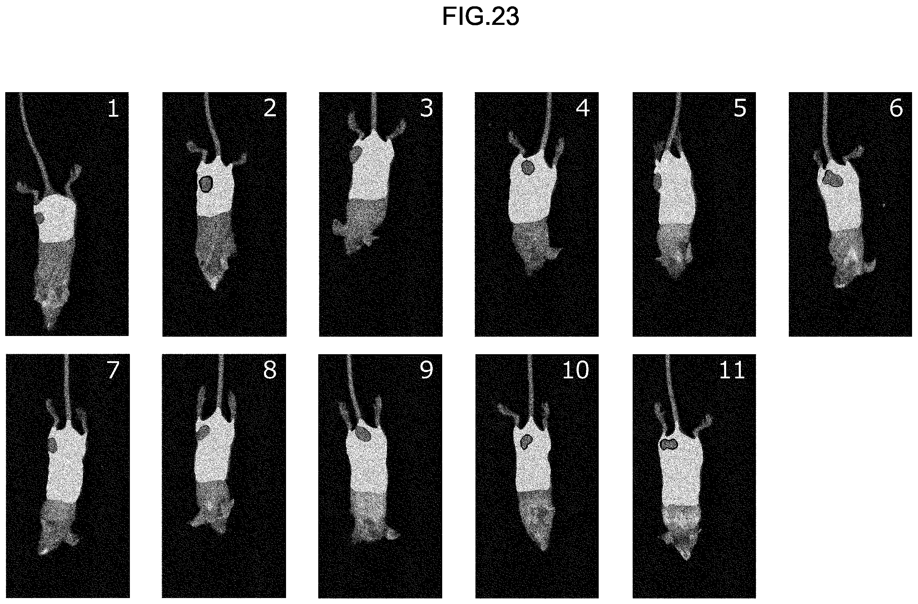

In addition, Figs. 22 to 27 show the results regarding a tumor-bearing mouse. Fig. 22 are images of a specimen. Fig. 23 is an image of training data. The white region in Fig. 23 is a normal region. The gray region present in the white region of Fig. 23 is a region corresponding to tumor tissue.

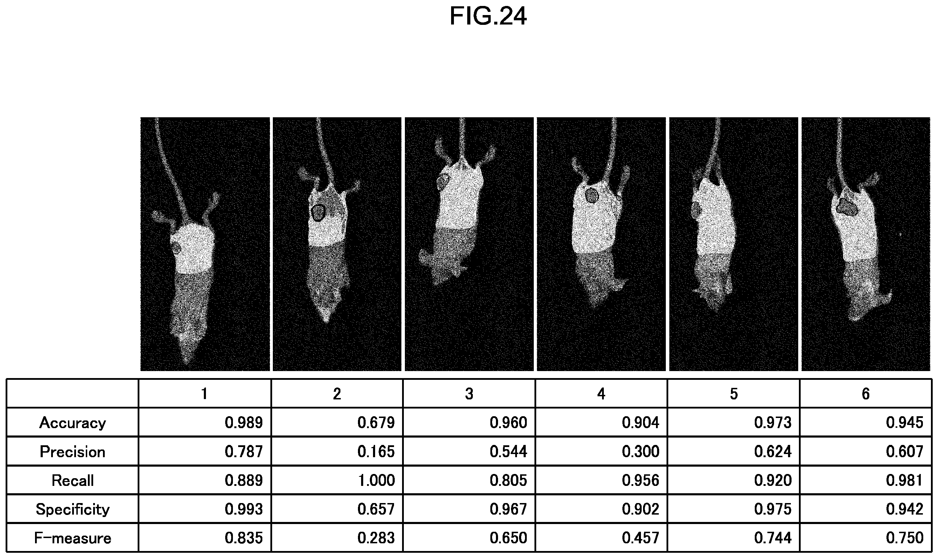

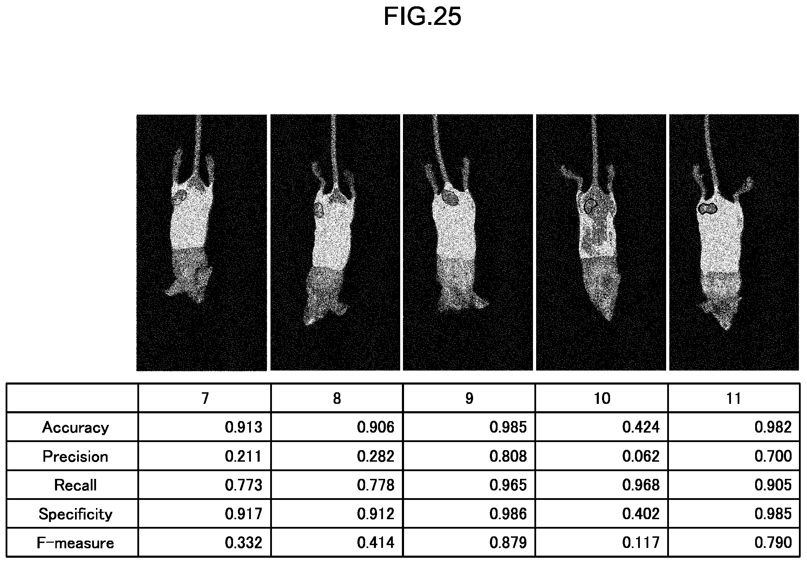

Figs. 24 and 25 are diagrams illustrating a region of a tumor estimated from an image obtained by irradiating a tumor-bearing mouse with light of 95 wavelengths. A white region is a region which is determined to be normal, and a gray region is a region which is determined to be a tumor. In addition, the table illustrated in the drawing represents the accuracy, precision, recall, specificity, and F-measure of the determination results for the pixels in each image.

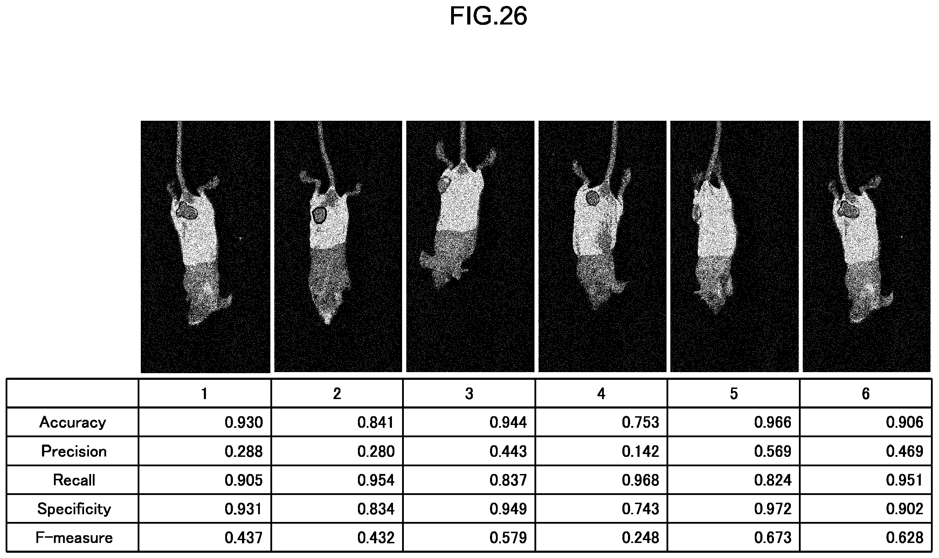

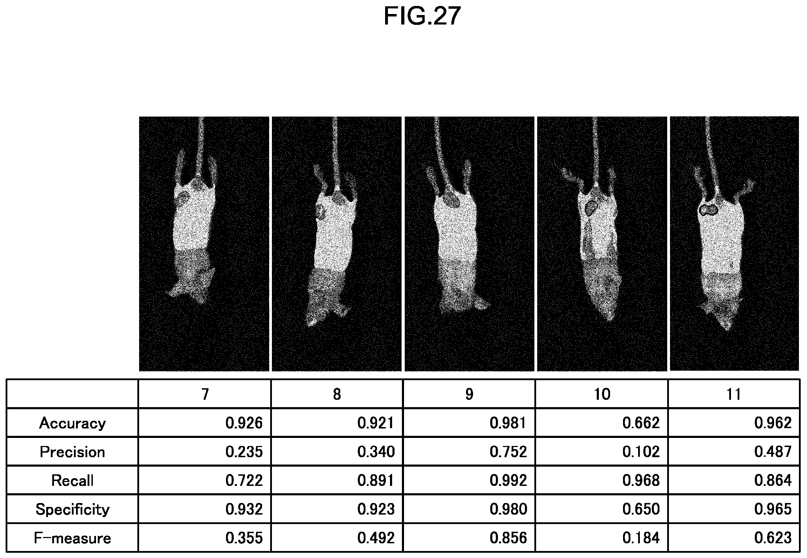

Figs. 26 and 27 are diagrams illustrating a region of a tumor estimated from an image obtained by irradiating a tumor-bearing mouse with light of four selected wavelengths. A white region is a region which is determined to be normal, and a gray region is a region which is determined to be a tumor. The table illustrated in the drawing represents the accuracy, precision, recall, specificity, and F-measure of the determination results for the pixels in each image.

When Fig. 24 and Fig. 25, and Fig. 26 and Fig. 27 are compared, it can be seen that there is not so much difference in the determination result. Accordingly, it may also be said that at least one of light having a wavelength of 1020 to 1140 [nm], light having a wavelength of 1140 to 1260 [nm], light having a wavelength of 1315 to 1430 [nm], and light having a wavelength of 1430 to 1535 [nm] is light having a wavelength useful for detecting human colorectal cancer.

Each of the above-described Examples is applicable to the foregoing embodiment, and can have a similar system configuration.

Note that, in the foregoing embodiments, each processing, which is executed as a result of the CPU reading software (a program), may be executed by various processors other than the CPU. Examples of the processor in this case include a programmable logic device (PLD) in which a circuit configuration can be changed after manufacturing a field-programmable gate array (FPGA) or the like, a dedicated electric circuit that is a processor having a circuit configuration exclusively designed for executing specific processing such as an application specific integrated circuit (ASIC), and the like. Moreover, each processing may be executed by one of these various processors, or may be executed by a combination of two or more processors of the same type or different types (for example, a plurality of FPGAs, a combination of a CPU and an FPGA, and so forth). Furthermore, the hardware structure of these various processors is, more specifically, an electric circuit in which circuit elements such as semiconductor elements are combined.

Note that the disclosure is not limited to or by the foregoing embodiment, rather, a variety of variations and applications are possible within a scope of the invention.

For example, in the embodiment, a case in which a GIST is detected using a neural network has been described as an example, but the disclosure is not limited thereto. For example, a GIST may be detected using the statistical model in the Example.

Further, in the embodiment, a case in which a gastrointestinal tract area is irradiated with first light representing light having a wavelength of 1050 to 1105 [nm], second light representing light having a wavelength of 1145 to 1200 [nm], third light representing light having a wavelength of 1245 to 1260 [nm], and fourth light representing light having a wavelength of 1350 to 1405 [nm] to acquire an image has been described as an example, but the disclosure is not limited thereto. A gastrointestinal tract area may be irradiated with at least one of these four light components to acquire an image. In addition, the same applies to each of the Examples, and an image may be acquired by irradiating an area of a living body with at least one of the light components.

Further, in the foregoing embodiment, a case in which a GIST is detected using light of four wavelengths has been described as an example, but the disclosure is not limited thereto. For example, a GIST may be detected using light of a plurality of wavelengths whose contribution amount in the Examples is equal to or greater than a predetermined threshold value. Furthermore, the same applies to the Examples, and a tumor may be detected using light of a plurality of wavelengths whose contribution amount is equal to or greater than a predetermined threshold value.

Moreover, in each of the Examples, light having a plurality of wavelengths is selected, but any light may be selected as long as the light has a wavelength, from the graph illustrated in the drawing, which can be regarded as useful.

## Claims

1. An image processing device (30), comprising:
an image acquisition unit (32) that acquires an image obtained by irradiating a gastrointestinal tract area of a living body with light having a wavelength of 1000 nm to 1500 nm; and
a determination unit (38) that inputs the image acquired by the image acquisition unit (32) to a learned model or a statistical model generated in advance for detecting, from the image, a gastrointestinal stromal tumor present in the gastrointestinal tract area, and that determines whether or not a gastrointestinal stromal tumor is present at each point in the image (Im) acquired by the image acquisition unit (32),
wherein the irradiating light includes:
first light representing light of a wavelength between 1050 and 1105 nm,
second light representing light of a wavelength between 1145 and 1200 nm,
third light representing light of a wavelength between 1245 and 1260 nm, and
fourth light representing light of a wavelength between 1350 and 1405 nm.

2. The image processing device (30) according to claim 1, wherein the learned model or the statistical model is a model generated in advance based on data in which an in-vivo image for training, and information indicating whether or not a gastrointestinal stromal tumor is present inside a gastrointestinal tract area appearing in the in-vivo image, are associated with each other.

3. The image processing device (30) according to claim 1 or 2, wherein the determination unit (38) inputs a pixel value of each pixel of the image (Im) acquired by the image acquisition unit (32) to the learned model or the statistical model, and determines whether or not a gastrointestinal stromal tumor is present for each pixel of the image acquired by the image acquisition unit.

4. An image processing program comprising instructions which, when the program is executed by a computer, cause the computer to carry out the method of claim 5.

5. An image processing method according to which a computer (20) executes processing, the processing comprising:
acquiring an image (Im) obtained by irradiating a gastrointestinal tract area of a living body with light having a wavelength of 1000 nm to 1500 nm; and
inputting the acquired image to a learned model or a statistical model generated in advance for detecting, from the image (Im), a gastrointestinal stromal tumor present in the gastrointestinal tract area, and determining whether or not a gastrointestinal stromal tumor is present at each point in the acquired image,
wherein the irradiating light includes:
first light representing light of a wavelength between 1050 and 1105 nm,
second light representing light of a wavelength between 1145 and 1200 nm,
third light representing light of a wavelength between 1245 and 1260 nm, and
fourth light representing light of a wavelength between 1350 and 1405 nm.

6. An endoscope device (12), comprising:
a light output unit (18B, 18C) that outputs first light representing light of a wavelength of 1050 to 1105 nm, second light representing light of a wavelength of 1145 to 1200 nm, third light representing light of a wavelength of 1245 to 1260 nm, and fourth light representing light of a wavelength of 1350 to 1405 nm; and
an imaging device (19; 18A) that captures an image (Im) when a gastrointestinal tract area of a living body is irradiated with light from the light output unit and that transmits the captured image to an image acquisition unit (32) of an image processing device (30) according to one of the claims 1 to 3.

7. An endoscope image processing system (1), comprising:
an endoscope device (12) according to claim 6; and
an image processing device (30) according to one of the claims 1 to 3.

## Patentansprüche

1. Bildverarbeitungsvorrichtung (30), umfassend
eine Bilderfassungseinheit (32), die ein Bild erfasst, welches durch Bestrahlen eines Magen-Darm-Trakt-Bereichs eines lebendigen Körpers mit Licht mit einer Wellenlänge von 1000 nm bis 1500 nm erhalten wird; und
eine Bestimmungseinheit (38), welche das durch die Bilderfassungseinheit (32) erfasste Bild in ein vorab erzeugtes trainiertes Modell oder statistisches Modell zum Detektieren eines in dem Magen-Darm-Trakt-Bereich vorhandenen gastrointestinalen Stromatumors aus dem Bild eingibt und die bestimmt, ob an jedem Punkt in dem durch die Bilderfassungseinheit (32) erfassten Bild (Im) ein gastrointestinaler Stromatumor vorhanden ist oder nicht,
wobei das Bestrahlungslicht Folgendes beinhaltet:
ein erstes Licht, welches Licht mit einer Wellenlänge zwischen 1050 und 1105 nm darstellt,
ein zweites Licht, das Licht mit einer Wellenlänge zwischen 1145 und 1200 nm darstellt,
ein drittes Licht, das Licht mit einer Wellenlänge zwischen 1245 und 1260 nm darstellt, und
ein viertes Licht, das Licht mit einer Wellenlänge zwischen 1350 und 1405 nm darstellt.

2. Bildverarbeitungsvorrichtung (30) gemäß Anspruch 1, wobei das trainierte Modell oder das statistische Modell ein vorab auf der Grundlage von Daten erzeugtes Modell ist, in denen ein In-vivo-Bild zum Trainieren und Informationen, die angeben, ob innerhalb eines in dem In-vivo-Bild erscheinenden Magen-Darm-Trakt-Bereichs ein gastrointestinaler Stromatumor vorhanden ist oder nicht, miteinander verknüpft sind.

3. Bildverarbeitungsvorrichtung (30) gemäß Anspruch 1 oder 2, wobei die Bestimmungseinheit (38) einen Pixelwert jedes Pixels des durch die Bilderfassungseinheit (32) erfassten Bildes (Im) in das trainierte Modell oder das statistische Modell eingibt und für jedes Pixel des durch die Bilderfassungseinheit erfassten Bildes bestimmt, ob ein gastrointestinaler Stromatumor vorhanden ist oder nicht.

4. Bildverarbeitungsprogramm, umfassend Anweisungen, die, wenn das Programm durch einen Computer ausgeführt wird, den Computer veranlassen, das Verfahren nach Anspruch 5 durchzuführen.

5. Bildverarbeitungsverfahren, gemäß dem ein Computer (20) eine Verarbeitung ausführt, wobei die Verarbeitung Folgendes umfasst:
Erfassen eines Bildes (Im), das durch Bestrahlen eines Magen-Darm-Trakt-Bereichs eines lebenden Körpers mit Licht mit einer Wellenlänge von 1000 nm bis 1500 nm erhalten wird; und
Eingeben des erfassten Bildes in ein vorab erzeugtes trainiertes Modell oder statistisches Modell zum Detektieren eines in dem Magen-Darm-Trakt-Bereich vorhandenen gastrointestinalen Stromatumors aus dem Bild (Im) und Bestimmen, ob an jedem Punkt in dem erfassten Bild ein gastrointestinaler Stromatumor vorhanden ist oder nicht,
wobei das Bestrahlungslicht Folgendes beinhaltet:
ein erstes Licht, das Licht mit einer Wellenlänge zwischen 1050 und 1105 nm darstellt,
ein zweites Licht, das Licht mit einer Wellenlänge zwischen 1145 und 1200 nm darstellt,
ein drittes Licht, das Licht mit einer Wellenlänge zwischen 1245 und 1260 nm darstellt, und
einviertes Licht, das Licht mit einer Wellenlänge zwischen 1350 und 1405 nm darstellt.

6. Endoskopvorrichtung (12), umfassend
eine Lichtausgabeeinheit (18B, 18C), die ein erstes Licht, das Licht mit einer Wellenlänge von 1050 bis 1105 nm darstellt, ein zweites Licht, das Licht mit einer Wellenlänge von 1145 bis 1200 nm darstellt, ein drittes Licht, das Licht mit einer Wellenlänge von 1245 bis 1260 nm darstellt, und ein viertes Licht, das Licht mit einer Wellenlänge von 1350 bis 1405 nm darstellt, ausgibt; und
eine Bildgebungsvorrichtung (19; 18A), die ein Bild (Im) aufnimmt, wenn ein Magen-Darm-Trakt-Bereich eines lebenden Körpers mit Licht von der Lichtausgabeeinheit bestrahlt wird, und die das aufgenommene Bild an eine Bilderfassungseinheit (32) einer Bildverarbeitungsvorrichtung (30) nach einem der Ansprüche 1 bis 3 überträgt.

7. Endoskopbildverarbeitungssystem (1), umfassend
eine Endoskopvorrichtung (12) gemäß Anspruch 6; und
eine Bildverarbeitungsvorrichtung (30) gemäß einem der Ansprüche 1 bis 3.

## Revendications

1. Dispositif de traitement d'image (30), comprenant :
une unité d'acquisition d'image (32) qui acquiert une image obtenue en irradiant une zone du tractus gastro-intestinal d'un corps vivant avec de la lumière ayant une longueur d'onde de 1000 nm à 1500 nm ; et
une unité de détermination (38) qui introduit l'image acquise par l'unité d'acquisition d'image (32) dans un modèle entraîné ou un modèle statistique généré à l'avance pour détecter, à partir de l'image, une tumeur stromale gastro-intestinale présente dans la zone du tractus gastro-intestinal, et qui détermine si une tumeur stromale gastro-intestinale est présente ou non en chaque point de l'image (Im) acquise par l'unité d'acquisition d'image (32),
dans lequel la lumière d'irradiation comprend :
une première lumière représentant une lumière d'une longueur d'onde comprise entre 1050 et 1105 nm,
une deuxième lumière représentant une lumière d'une longueur d'onde comprise entre 1145 et 1200 nm,
une troisième lumière représentant une lumière d'une longueur d'onde comprise entre 1245 et 1260 nm, et
une quatrième lumière représentant une lumière d'une longueur d'onde comprise entre 1350 et 1405 nm.

2. Le dispositif de traitement d'image (30) selon la revendication 1, dans lequel le modèle entraîné ou le modèle statistique est un modèle généré à l'avance sur la base de données dans lesquelles une image in vivo destinée à l'entraînement et des informations indiquant si une tumeur stromale gastro-intestinale est présente ou non à l'intérieur d'une zone du tractus gastro-intestinal apparaissant dans l'image in vivo sont associées les unes aux autres.

3. Le dispositif de traitement d'image (30) selon la revendication 1 ou 2, dans lequel l'unité de détermination (38) introduit une valeur de pixel de chaque pixel de l'image (Im) acquise par l'unité d'acquisition d'image (32) dans le modèle entraîné ou le modèle statistique, et détermine, pour chaque pixel de l'image acquise par l'unité d'acquisition d'image, si une tumeur stromale gastro-intestinale est présente ou non.

4. Un programme de traitement d'image comprenant des instructions qui, lorsque le programme est exécuté par un ordinateur, amènent l'ordinateur à mettre en œuvre le procédé selon la revendication 5.

5. Un procédé de traitement d'image selon lequel un ordinateur (20) exécute un traitement, le traitement comprenant :
l'acquisition d'une image (Im) obtenue en irradiant une zone du tractus gastro-intestinal d'un corps vivant avec de la lumière ayant une longueur d'onde de 1000 nm à 1500 nm ; et
l'introduction de l'image acquise dans un modèle entraîné ou un modèle statistique généré à l'avance pour détecter, à partir de l'image (Im), une tumeur stromale gastro-intestinale présente dans la zone du tractus gastro-intestinal, et la détermination du fait qu'une tumeur stromale gastro-intestinale est présente ou non en chaque point de l'image acquise,
dans lequel la lumière d'irradiation comprend :
une première lumière représentant une lumière d'une longueur d'onde comprise entre 1050 et 1105 nm,
une deuxième lumière représentant une lumière d'une longueur d'onde comprise entre 1145 et 1200 nm,
une troisième lumière représentant une lumière d'une longueur d'onde comprise entre 1245 et 1260 nm, et
une quatrième lumière représentant une lumière d'une longueur d'onde comprise entre 1350 et 1405 nm.

6. Un dispositif endoscopique (12), comprenant :
une unité de sortie de lumière (18B, 18C) qui émet une première lumière représentant une lumière d'une longueur d'onde de 1050 à 1105 nm, une deuxième lumière représentant une lumière d'une longueur d'onde de 1145 à 1200 nm, une troisième lumière représentant une lumière d'une longueur d'onde de 1245 à 1260 nm, et une quatrième lumière représentant une lumière d'une longueur d'onde de 1350 à 1405 nm ; et
un dispositif d'imagerie (19 ; 18A) qui capture une image (Im) lorsqu'une zone du tractus gastro-intestinal d'un corps vivant est irradiée avec de la lumière provenant de l'unité de sortie de lumière et qui transmet l'image capturée à une unité d'acquisition d'image (32) d'un dispositif de traitement d'image (30) selon l'une des revendications 1 à 3.

7. Un système de traitement d'image endoscopique (1), comprenant :
un dispositif endoscopique (12) selon la revendication 6 ; et
un dispositif de traitement d'image (30) selon l'une des revendications 1 à 3.
